# EUROPEAN PATENT SPECIFICATION

(11) **EP 3 763 394 B1**
(45) Date of publication and mention of the grant of the patent: **08.11.2023**
(21) Application number: 20193861.0
(22) Date of filing: 02.03.2016
(51) Int. Cl.: A61K 51/08, A61K 51/04, C07B 59/00, A61K 103/00

(54) **METHOD FOR LABELING A PROSTATE-SPECIFIC MEMBRANE ANTIGEN LIGAND WITH A RADIOACTIVE ISOTOPE**
VERFAHREN ZUR MARKIERUNG EINES PROSTATA-SPEZIFISCHEN MEMBRAN ANTIGEN LIGANDEN MIT EINEM RADIOAKTIVEN ISOTOP
PROCÉDÉ DE MARQUAGE D'UN LIGAND D'ANTIGÈNE MEMBRANAIRE SPÉCIFIQUE DE LA PROSTATE AVEC UN ISOTOPE RADIOACTIF

(30) Priority: 03.03.2015 IL 23752515
(43) Date of publication of application: 13.01.2021
(62) Divisional of application: 16158323.2
(73) Proprietor: Isotopia Molecular Imaging Ltd, 49277 Petach Tikva (IL)
(72) Inventor: SHALOM, Eli, 7579517 Rishon LeZyon (IL)
(74) Representative: ABG Intellectual Property Law, S.L.

(56) References cited:
- MATTHIAS EDER ET AL: "Novel Preclinical and Radiopharmaceutical Aspects of [68Ga]Ga-PSMA-HBED-CC: A New PET Tracer for Imaging of Prostate Cancer", PHARMACEUTICALS, vol. 7, no. 7, 30 June 2014 (2014-06-30), pages 779-796, XP055280737, DOI: 10.3390/ph7070779
- MATTHIAS EDER ET AL: "68 Ga-Complex Lipophilicity and the Targeting Property of a Urea-Based PSMA Inhibitor for PET Imaging", BIOCONJUGATE CHEMISTRY, vol. 23, no. 4, 18 April 2012 (2012-04-18) , pages 688-697, XP055043532, ISSN: 1043-1802, DOI: 10.1021/bc200279b
- KRATOCHWIL CLEMENS ET AL: "[177Lu]Lutetium-labelled PSMA ligand-induced remission in a patient with metastatic prostate cancer", EUROPEAN JOURNAL OF NUCLEAR MEDICINE AND MOLECULAR IMAGING, SPRINGER VERLAG, HEIDELBERG, DE, vol. 42, no. 6, 9 January 2015 (2015-01-09), pages 987-988, XP035444727, ISSN: 1619-7070, DOI: 10.1007/S00259-014-2978-1 [retrieved on 2015-01-09]
- ARCHANA MUKHERJEE ET AL: "Development of Single Vial Kits for Preparation of 68Ga-Labelled Peptides for PET Imaging of Neuroendocrine Tumours", MOLECULAR IMAGING & BIOLOGY, vol. 16, no. 4, 30 January 2014 (2014-01-30), pages 550-557, XP055289358, Boston ISSN: 1536-1632, DOI: 10.1007/s11307-014-0719-2
- THOMAS EBENHAN ET AL: "Development of a Single Vial Kit Solution for Radiolabeling of 68Ga-DKFZ-PSMA-11 and Its Performance in Prostate Cancer Patients", MOLECULES, vol. 20, no. 8, 14 August 2015 (2015-08-14), pages 14860-14878, XP055289112, DOI: 10.3390/molecules200814860
- SATPATI DRISHTY ET AL: "Convenient Preparation of [68Ga]DKFZ-PSMA-11 Using a Robust Single-Vial Kit and Demonstration of Its Clinical Efficacy", MOLECULAR IMAGING & BIOLOGY, ELSEVIER, BOSTON, vol. 18, no. 3, 16 March 2016 (2016-03-16) , pages 420-427, XP035936489, ISSN: 1536-1632, DOI: 10.1007/S11307-016-0943-Z [retrieved on 2016-03-16]
- WATERHOUSE N N ET AL: "Otto: a 4.04 GBq (109 mCi) <68>Ge/<68>Ga generator, first of its kind - extended quality control and performance evaluation in the clinical production of [<68>Ga]Ga-PSMA-11", 1 December 2020 (2020-12-01), EJNMMI RADIOPHARMACY AND CHEMISTRY 20201201 SPRINGER SCIENCE AND BUSINESS MEDIA DEUTSCHLAND GMBH CHE, VOL. 5, NR. 1 ISSN: 2365-421X
- MCCARTHY M ET AL: "Comparison of PSMA-HBED and PSMA-I&T as diagnostic agents in prostate carcinoma", EUROPEAN JOURNAL OF NUCLEAR MEDICINE AND MOLECULAR IMAGING 20170801 SPRINGER BERLIN HEIDELBERG DEU, vol. 44, no. 9, 1 August 2017 (2017-08-01) , pages 1455-1462, ISSN: 1619-7070
- ANONYMOUS: "Galli Eo Product Info", , Retrieved from the Internet: URL:https://www.ire.eu/en/our-activities/i re-elit/galli-eo> [retrieved on 2023-01-16]
- ANONYMOUS: "Summary of product characteristics Galliapharm", HEALTH PRODUCT REGULATORY AUTHORITIES, 25 January 2021 (2021-01-25),
- FORRER F ET AL: "Treatment with<177>Lu-DOTATOC of patients with relapse of neuroendocrine tumors after treatment with<90>Y-DOTATOC", JOURNAL OF NUCLEAR MEDICINE 2005 US, vol. 46, no. 8, 2005, pages 1310-1316, ISSN: 0161-5505

## Description

### FIELD OF THE INVENTION

The present invention relates to a method for labeling a prostate-specific membrane antigen (PSMA) ligand with a radioactive isotope. Radiolabeled PSMA ligands prepared by this method can be used for both imaging and therapy purposes.

### BACKGROUND OF THE INVENTION

Prostate cancer is the second most frequent cancer and the fifth leading cause of cancer death in men worldwide. One of the key issues in treatment of this cancer is the detection of recurrent disease or metastases. In view of the fact that prostate cancer cells tend to spread to other organs such as the bones and lymph nodes, early detection of the primary prostate cancer tumor is highly desirable to prevent metastases formation, and furthermore, effective treatment strategies for prostate cancer metastases are urgently needed. Comprehensibly, targeting of prostate cancer or metastases thereof is a demanding task in the field of molecular imaging with positron emission tomography (PET), and targeted internal radiation therapy is a major challenge for all conventional imaging modalities. Although choline-based PET/CT is widely used for this purpose, there have been numerous studies reporting low sensitivity and specificity, especially when low PSMA levels are present in the inspected subject. Improved imaging of prostate cancer is thus necessary.

The cell surface protein PSMA, also known as glutamate carboxypeptidase II (GCPII), is a membrane-type zinc protease that catalyzes the hydrolysis of *N*-acetyl-L-aspartyl-L-glutamate (NAAG) into the corresponding N-acetyl-L-aspartate (NAA) and L-glutamate. PSMA was found to be significantly overexpressed in prostate cancer cells, and in particular in poorly differentiated, metastatic and hormone-refractory carcinomas, compared with other PSMA expressing tissues (e.g., the brain, kidney, salivary gland and the small intestine) where about 10-80 fold lower PSMA levels were detected. These findings make PSMA an ideal biological target for high quality PET imaging of prostate cancer as well as for prostate cancer therapy. Accordingly, different techniques have been developed for testing the level of PSMA, and radiolabeled PSMA tracers/ligands have been developed for imaging and targeted therapy purposes.

Based on the chemical structure of NAAG, several urea-based PSMA ligands (tracers; inhibitors), e.g., glutamate-urea-glutamate-based peptides bearing a 2-[3-(1,3-dicarboxypropyl)-ureido]pentanedioic acid (DUPA) moiety, were developed. Those molecules showed high affinity and specific binding to PSMA, as demonstrated in binding studies using PSMA expressing LNCaP cell lines, and differ mainly in the selection of the chelation agent used for the complexation of the desired radionuclide. Moreover, since the clearance of those molecules from the blood circulation is quite rapid, such urea-based PSMA inhibitors are considered as ideal biological PSMA tracers.

Different methods have been developed for labeling PSMA ligands with ⁶⁸Ga to thereby obtain labeled PSMA ligands that can be used in PET imaging and in therapy. Initial experience with PET/CT using the ⁶⁸Ga-labeled urea-based PSMA ligand 4,6,12,19-tetraazadocosane-1,3,7-tricarboxylic acid, 22-[3-[[[2-[[[5-(2-carboxyethyl)-2-hydroxyphenyl]methyl](carboxy-methyl)amino]ethyl](carboxymethyl) amino]methyl]-4-hydroxyphenyl]-5,13,20-trioxo-(3S,7S)-trifluoroacetate salt, herein identified as **DKFZ-PSMA-11** (see Table 1), as a prostate cancer cell tracer, suggests that this tracer can detect prostate cancer relapses and metastases with high contrast by specifically binding to the extracellular domain of PSMA on prostate cancer cells, followed by internalization. Initial clinical trials using ⁶⁸Ga-labelled **DKFZ-PSMA-11** have shown that this PSMA tracer detects prostate cancer relapses and metastases with higher contrast compared to the commonly used ¹⁸F-choline tracer. Moreover, even at low PSMA levels, PET/CT images obtained when using this tracer showed more prostate cancer lesions compared to PET/CT images obtained when ¹⁸F-choline was used.

In radio-metal therapy approaches, the application of ⁹⁰Y and ¹⁷⁷Lu is favored. The use of ⁹⁰Y (Eβₘₐₓ=2.3 MeV, t_{½}=64 h) is more appropriate in the treatment of larger tumor lesions, while ¹⁷⁷Lu (Eβₘₐₓ=0.5 MeV, t_{½}=6.7 d) is more suitable for the treatment of smaller lesions and metastases, accompanied by a minimization of kidney dose in comparison to the application of ⁹⁰Y labeled peptides. Moreover, due to the contemporary beta- and gamma-emission, ¹⁷⁷Lu is a useful diagnostic tool for scintigraphy of tumoral uptake.

Several developments based on the chelator diethylenetriaminepenta-acetic acid (DTPA), lead to the promising cyclohexyl substituted analogue, cyclohexyl-diethylene triamine pentaacetic acid (CHX-A"-DTPA), which showed high stability *in vivo*, and radiolabelling with ⁹⁰Y was achieved under mild conditions (pH=6 at room temperature). Consequently, several studies were conducted involving the synthesis of the PSMA ligand cyclohexyl-diethylene triamine pentaacetic acid-(5S,8S,22S,26S)-1-amino-5,8-dibenzyl-4,7,10,19,24-pentaoxo-3,6,9,18,23,25-hexaazaoctacosane-22,26,28-tricarboxylic acid (CHX-A"-DTPA-DUPA-Pep), labeled with ⁶⁸Ga, ¹⁷⁷Lu or ⁹⁰Y. In cell research with PSMA-positive androgen-insensitive LNCaP-C4-2 cells, this PSMA ligand showed KD values of ≤14.67±1.95 nM, indicating high biological activity towards PSMA. Other studies using the PSMA ligand (SS,8S,22S,26S)-1-amino-5,8-dibenzyl-4,7,10,19,24-pentaoxo-3,6,9,18,23,25-hexaazaoctacosane-22,26,28-tricarboxylic acid (DUPA-Pep), conjugated with 1,4,7,10-tetraazacyclododecane-*N*,*N′*,*Nʺ*,*N‴*-tetraacetic acid (DOTA) and the same cells, revealed a high PSMA-affinity with a KD of 21.6±0.4 nM.

Currently, DOTA is the mostly used chelator for the complexation of radio-metals such as ⁶⁸Ga, ¹⁷⁷Lu and ⁹⁰Y, for both diagnostic and therapeutic application. However, labeling reactions using DOTA as the chelating agent are usually carried out at high temperatures under acidic conditions and require long reaction times, which might result in decomposition of the PSMA ligand, the radioactive isotope, or both.

Today, a fully automated synthesis module is used for radio-synthezising a radio-labled PSMA tracer for clinical applications such as imaging and therapy. However, the process performed by this module is time consuming and requires expensive dedicated machinery and equipment, which occupy a large space. Moreover, this process results with a substantive amount of unbound radio-isotope and therefore requires an additional dedicated purification step for the removal of said unbound radio-isotope.

In view of the relatively short half-life of the ⁶⁸Ga isotope (about 68 minutes only), the fully automated synthesis module currently used for radio-synthezising ⁶⁸Galabeled **DKFZ-PSMA-11**, for imaging purposes, is further combined with a ⁶⁸Ge/⁶⁸Gagenerator. This automated synthesis module has to be quarantined in order to prevent radiation exposure of the close environment. Said quarantine is predominantly done by using a dedicated protection hot-cell, and the entire construction is usually placed at a dedicated room. The entire system, i.e., the module, the hot-cell and the required space, is highly expensive and requires constant and high maintenance costs. In addition, since the duration of the procedure for radiolabeling **DKFZ-PSMA-11** using the automated module is about 20-30 minutes, the radiolabeled PSMA tracer obtained thereby has to be used, i.e., administered to the subject, immediately before it is no longer effective.

Therefore, there is an unmet need for developing new, simple, fast and cost efficient ways for radiolabeling PSMA tracers.

### SUMMARY OF INVENTION

It has now been found, in accordance with the present invention, that highlyefficient radiolabeling of urea-based PSMA ligands, such as **DKFZ-PSMA-11**, with the radioactive isotope ⁶⁸Ga, can be carried out in a fast, simple and safe manner which renders the use of the current automated synthesis module, and the requirements for the whole protecting constructions associated therewith, redundant. As shown herein, such a process is applicable for radioactive isotopes other than ⁶⁸Ga as well, such as ¹⁷⁷Lu, or ⁹⁰Y.

In one aspect, the present invention thus relates to a "shake & bake" method for labeling a PSMA ligand with a radioactive isotope selected from ⁶⁸Ga, ¹⁷⁷Lu, or ⁹⁰Y, said method comprising the steps of: (i) providing a reaction vial containing a predetermined amounts of a sodium-based buffering agent and said PSMA ligand, both in dried form; (ii) adding a solution of said radioactive isotope in a predetermined amount of HCl having a predetermined molarity to said reaction vial, thus obtaining a solution of said PSMA ligand and said radioactive isotope in said HCl; (iii) mixing the solution obtained in (ii) and then incubating it for a sufficient period of time, thus reacting said PSMA ligand with said radioactive isotope to thereby obtain said PSMA ligand labeled with said radioactive isotope, wherein at least 90%, preferably 95%, of said radioactive isotope in said solution is bound to said PSMA ligand; and (iv) optionally adjusting the pH of the solution in said reaction vial to a pH compatible with physiological conditions by adding a sodium-based buffering agent,
wherein said PSMA ligand is of the general formula I:

**I** R₁-CO-R₂-L-B-X

wherein
B is a chelating agent capable of coordinating with said radioactive isotope;
X is absent or is of the formula -L'-R₂'-CO-R₁';
L and L' each independently is absent or a linker;
R₁ and R₁', if present, each is a glutamic acid residue linked via the amino group thereof to the adjacent -CO- group; and
R₂ and R₂', if present, each is a glutamic acid residue or a lysine residue linked via an amino group thereof to the adjacent -CO- group.

In one particular embodiment exemplified herein, the method of the invention is used for labeling a PSMA ligand with ⁶⁸Ga, wherein said PSMA ligand is **DKFZ-PSMA-11**, i.e., a compound of the general formula I, wherein X is absent; R₁ is a glutamic acid residue linked via its amino group to the adjacent -CO- group; R₂ is a lysine residue linked via its α-amino group to the adjacent -CO- group; L is 6-aminohexanoic acid; and B is HBED-CC, i.e., *N*,*N'*-bis-[2-hydroxy-5-(carboxyethyl) benzyl]ethylene-diamine-*N*,*N'*-diacetic acid, linked via a carboxylic group thereof to the amino group of the 6-aminohexanoic acid.

The labeled PSMA ligand prepared according to the method of the invention may be used for either imaging or treatment of a primary prostate cancer or metastases thereof, wherein PSMA ligands labeled with ⁶⁸Ga are typically used for imaging, and PSMA ligands labeled with either ¹⁷⁷Lu or ⁹⁰Y are typically used for treatment. In particular embodiments, the method of the invention thus further comprises the step of administering the solution obtained in step (iv) to a subject, i.e., a mammal in general or human in particular, for imaging or for treatment of prostate cancer or metastases thereof.

A PSMA ligand labeled with a radioactive isotope by the method defined above, wherein said radioactive isotope is ⁶⁸Ga, may be used in imaging of prostate cancer or metastases thereof; and such a labeled PSMA ligand, wherein said radioactive isotope is ¹⁷⁷Lu or ⁹⁰Y, may be used in treatment of prostate cancer or metastases thereof.

A kit for carrying out the method defined above is also disclosed, more particularly, a kit comprising: (i) a disposable reaction vial containing a predetermined amounts of both a sodium-based buffering agent and a PSMA ligand of the general formula I as defined above, both in dried form; or two disposable reaction vials, wherein one of said reaction vials contains said sodium-based buffering agent and the other of said reaction vials contains said PSMA ligand, both in dried form; and (ii) instructions for optionally adding said sodium-based buffering agent to the reaction vial containing said PSMA ligand, and for labeling said PSMA ligand with a radioactive isotope. The kit may further comprise (i) a vial containing a predetermined amount of HCl having a molarity in a range of 0.05 to 0.1N for eluting said radioactive isotope from a radioactive isotope generator; and (ii) a vial containing a sodium-based buffering agent for pH adjustment. This kit does not form part of the invention.

### DETAILED DESCRIPTION OF THE INVENTION

The present invention describes new methods for radiolabeling PSMA ligands with suitable radioactive isotopes, such as ⁶⁸Ga, ⁹⁰Y and ¹⁷⁷Lu, under mild reaction conditions (e.g., room temperature) and for short periods of time (e.g., 3 to 30 minutes), while still obtaining quantitative radiochemical yields, wherein said radiolabeled PSMA isotope is suitable for the diagnosis or therapy of prostate cancer. Accordingly, the present invention provides methods for radiolabeling PSMA, which are both simple and fast, and require neither sophisticated nor expensive equipment as required by the automated synthesis module currently available, such as the "hot-cell". Moreover, since the method of the invention enables over 95% binding of the radiaoactive isotop to said PSMA ligand, it eliminates the need for a step of removing unbound isotop from the reaction vial, which is an essential step of the presently known labeling methods, involving, e.g., using a designated cartridge of column for said unbound isotop removal.

In one aspect, the present invention thus provides a method for labeling a PSMA ligand with a radioactive isotope selected from ⁶⁸Ga, ¹⁷⁷Lu, or ⁹⁰Y, said method consisting essentially of the steps of: (i) providing a reaction vial containing a predetermined amounts of a sodium-based buffering agent and said PSMA ligand, both in dried form, i.e., either lyophilized or spray dried; (ii) adding a solution of said radioactive isotope in a predetermined amount of HCl having a predetermined molarity to said reaction vial, thus obtaining a solution of said PSMA ligand and said radioactive isotope in said HCl; (iii) mixing the solution obtained in (ii) and then incubating it for a sufficient period of time, thus reacting said PSMA ligand with said radioactive isotope to thereby obtain said PSMA ligand labeled with said radioactive isotope, wherein at least 90%, preferably 95%, of said radioactive isotope in said solution is bound to said PSMA ligand; and (iv) optionally adjusting the pH of the solution in said reaction vial to a pH compatible with physiological conditions by adding a sodium-based buffering agent,
wherein said PSMA ligand is of the general formula I:

**I** R₁-CO-R₂-L-B-X

wherein B is a chelating agent capable of coordinating with said radioactive isotope; X is absent or is of the formula -L'-R₂'-CO-R₁'; L and L' each independently is absent or a linker; R₁ and R₁', if present, each is a glutamic acid residue linked via the amino group thereof to the adjacent -CO- group; and R₂ and R₂', if present, each is a glutamic acid residue or a lysine residue linked via an amino group thereof to the adjacent -CO- group.

The terms "PSMA ligand", "PSMA tracer" and "PSMA inhibitor", used herein interchangeably, refer to a compound of the general formula I defined above, i.e., a compound comprising (a) an urea core formed by linking the said two amino acid residues via their amino groups to a carbonyl group, and capable of interacting thus inhibiting the PSMA enzyme; and (b) a chelating agent capable of coordinating with a radioactive isotope selected from ⁶⁸Ga, ¹⁷⁷Lu, or ⁹⁰Y, and linked to said urea core optionally via a linker linked to one of said amino acid residues. Particular PSMA ligands described herein are those having an urea core formed by linking glutamic acid and lysine residues, each via its α-amino group, to a carbonyl group, and a chelating agent linked to the side chain amino group of the lysine residue via a linker consisting of an amino acid or a dipeptide.

The term "amino acid" as used herein refers to an organic compound comprising both amine and carboxylic acid functional groups, which may be either a natural or synthetic, i.e., non-natural, amino acid in its both L- and D-stereoisomers. The twenty two natural amino acids are aspartic acid (Asp), tyrosine (Tyr), leucine (Leu), tryptophan (Trp), arginine (Arg), valine (Val), glutamic acid (Glu), methionine (Met), phenylalanine (Phe), serine (Ser), alanine (Ala), glutamine (Gln), glycine (Gly), proline (Pro), threonine (Thr), asparagine (Asn), lysine (Lys), histidine (His), isoleucine (Ile), cysteine (Cys), selenocysteine (Sec), and pyrrolysine (Pyl). A non-natural amino acid is any amino acid, modified amino acid and/or an analog thereof, that is not one of those natural amino acids, wherein non-limiting examples of non-natural amino acids include diaminopropionic acid (Dap), diaminobutyric acid (Dab), ornithine (Orn), aminoadipic acid, β-alanine, α-aminohexanoic acid (Ahx), 6-aminohexanoic acid, 8-aminooctanoic acid, 1-naphthylalanine (1Nal), 2-naphthylalanine (2Nal), 3-(1-naphthyl)alanine, 3-(2-naphthyl)alanine, 4-(aminomethyl)cyclohexane carboxylic acid (Amc), γ-aminobutiric acid (GABA), 3-(aminomethyl) benzoic acid, *p*-ethynyl-phenylalanine, *p*-propargly-oxy-phenylalanine, *m*-ethynyl-phenylalanine, *p-*bromophenylalanine, *p*-iodophenylalanine, *p*-azidophenylalanine, *p-*acetylphenylalanine, azidonorleucine, 6-ethynyl-tryptophan, 5-ethynyl-tryptophan, 3-(6-chloroindolyl)alanine, 3-(6-bromoindolyl)alanine, 3-(5-bromoindolyl) alanine, azidohomoalanine, *p*-chlorophenylalanine, α-aminocaprylic acid, O-methyl-L-tyrosine, N-acetylgalactosamine-α-threonine, and N-acetylgalactosamine-α-serine. The term "amino acid residue" refers to a residue of an amino acid results from removal of a hydrogen atom(s) from either an amine or carboxylic acid group thereof, or from both amine and carboxylic groups thereof.

The term "peptide" as used herein refers to a chain of 2-8 amino acid monomers linked by peptide bonds, i.e., the covalent bond formed when a carboxyl group of one amino acid reacts with an amino group of another. Particular such peptides are those consisting of 2-6 amino acid residues, more particularly 2, 3, or 4 amino acid residues, i.e., dipeptides, tripeptides or tetrapeptides, respectively.

The term "alkylene" as used herein refers to a straight or branched divalent hydrocarbon radical having 1-8 carbon atoms and includes, e.g., methylene, ethylene, propylene, butylene, 2-methylpropylene, pentylene, 2-methylbutylene, hexylene, 2-methylpentylene, 3-methylpentylene, 2,3-dimethylbutylene, heptylene, octylene, and the like. Preferred are (C₁-C₆)alkylene, (C₁-C₄)alkylene, and (C₂-C₄)alkylene. The term "alkenylene" typically means straight or branched divalent hydrocarbon radicals having 2-8 carbon atoms and one or more double bond such as, without limiting, ethenylene, propenylene, 1- and 2-butenylene, 1- and 2-pentenylene, 1-, 2- and 3-hexenylene, heptenylene, octenylene, and the like. The terms "alkynylene" typically means straight or branched divalent hydrocarbon radicals having 2-8 carbon atoms and one or more triple bond such as, without limiting, ethynylene, propynylene, 1- and 2-butynylene, 1- and 2-pentynylene, 1-, 2- and 3-hexynylene, heptynylene, octynylene, and the like.

The PSMA ligand being labeled according to the method of the present invention has the general formula I as defined above, wherein R₁ and R₁', if present, each is a glutamic acid residue linked via the amino group thereof to the adjacent -CO-group.

The PSMA ligand being labeled according to the method of the present invention has the general formula I as defined above, wherein R₂ and R₂', if present, each is a glutamic acid residue linked via the amino group thereof to the adjacent -CO-group, or a lysine residue linked via either the α-amino or side chain amino group thereof to the adjacent -CO- group. In particular such embodiments, R₂ and R₂', if present, each is a lysine residue linked via its α-amino group to the adjacent -CO-group.

In certain embodiments, the PSMA ligand being labeled according to the method of the present invention has the general formula I as defined above, wherein L and L' each independently is absent or a linker selected from an amino acid residue forming a peptide bond with R₂ or R₂', respectively, a peptide moiety consisting of 2-8, e.g., 2, 3, 4, 5, or 6, amino acid residues and forming a peptide bond with R₂ or R₂', respectively, (C₁-C₈)alkylene, (C₂-C₈)alkenylene or (C₂-C₈)alkynylene, wherein said (C₁-C₈)alkylene, (C₂-C₈)alkenylene and (C₂-C₈)alkynylene is optionally substituted with one or more, e.g., 1, 2, or 3, groups each independently is selected from halogen, - COR₃, -COOR₃, -OCOOR₃, -OCON(R₃)₂, -CN, -NO₂, -SR₃, -OR₃, -N(R₃)₂, -CON(R₃)₂, -SO₂R₃, -SO₃H, or -S(=O)R₃, and further optionally interrupted by one or more, e.g., 1, 2, or 3, identical or different heteroatoms selected from S, O or N, and/or at least one group, e.g., 1, 2, or more groups, selected from -NH-CO-, -CO-NH-, -N(C₁-C₁alkyl)-, wherein R₃ each independently is selected from hydrogen, or -(C₁-C₈)alkyl.

In certain particular such embodiments, L and L' each independently is a linker selected from an amino acid residue, or a peptide moiety consisting of 2-6 amino acid residues, wherein said amino acid each independently is 6-aminohexanoic acid, 8-aminooctanoic acid, 1-naphthylalanine (1Nal), 2-naphthylalanine (2Nal), or 4-(aminomethyl) cyclohexane carboxylic acid (Amc). Preferred such embodiments are those wherein said linker is 6-aminohexanoic acid, 8-aminooctanoic acid, 1Nal-Amc, 2Nal-Amc, Amc-1Nal, or Amc-2Nal.

In other particular such embodiments, L and L' each independently is a linker selected from (C₁-C₈)alkylene, (C₂-C₈)alkenylene or (C₂-C₈)alkynylene, optionally substituted with one or more groups each independently is selected from halogen, -COH, -COOH, -OCOOH, -OCONH₂, -CN, -NO₂, -SH, -OH, -NH₂, -CONH₂, -SO₂H, -SO₃H, or -S(=O)H, and further optionally interrupted by one or more identical or different heteroatoms selected from S, O or N, and/or at least one group selected from -NH-CO-, -CO-NH-, -N(C₁-C₈alkyl)-.

The chelating agent HBED-CC is a rarely used acyclic complexing agent especially allowing efficient radiolabelling with, e.g., ⁶⁸Ga even at ambient temperature. Accordingly, in certain embodiments, the PSMA ligand being labeled according to the method of the present invention has the general formula I as defined above, wherein said chelating agent being capable of coordinating with said radioactive isotope is HBED-CC, forming amide bond with either the linker L, if present, or R₂, and when X is present, forming another amide bond with either the linker L', if present, or R₂'.

An alternative chelating agent, widely used for the complexation of various radioactive isotopes such as ⁶⁸Ga, ¹⁷⁷Lu and ⁹⁰Y, for both diagnostic and therapeutic application, is DOTA. In other embodiments, the PSMA ligand being labeled according to the method of the present invention has the general formula I as defined above, wherein said chelating agent is thus DOTA, forming amide bond with either the linker L, if present, or R₂, and when X is present, forming another amide bond with either the linker L', if present, or R₂'.

In certain embodiments, the PSMA ligand being labeled according to the method of the present invention has the general formula I as defined above, wherein R₁ and R₁', if present, are a glutamic acid residue; R₂ and R₂', if present, are a glutamic acid residue or a lysine residue preferably linked via its α-amino group to the adjacent -CO-group; L and L' each independently is absent or a linker selected from an amino acid residue forming a peptide bond with R₂ or R₂', respectively, a peptide moiety consisting of 2-8 amino acid residues and forming a peptide bond with R₂ or R₂', respectively, (C₁-C₈)alkylene, (C₂-C₈)alkenylene or (C₂-C₈)alkynylene, wherein said (C₁-C₈)alkylene, (C₂-C₈)alkenylene and (C₂-C₈)alkynylene is optionally substituted with one or more groups each independently is selected from halogen, -COR₃, -COOR₃, -OCOOR₃, - OCON(R₃)₂, -CN, -NO₂, -SR₃, -OR₃, -N(R₃)₂, -CON(R₃)₂, -SO₂R₃, -SO₃H, or -S(=O)R₃, and further optionally interrupted by one or more identical or different heteroatoms selected from S, O or N, and/or at least one group selected from -NH-CO-, -CO-NH-, - N(C₁-C₈alkyl)-, wherein R₃ each independently is selected from hydrogen, or -(C₁-C₈)alkyl; said radioactive isotope is ⁶⁸Ga, ¹⁷⁷Lu, or ⁹⁰Y; and said chelating agent is HBED-CC or DOTA, forming amide bond with either L, if present, or R₂, and when X is present, forming another amide bond with either L', if present, or R₂'. In particular such embodiments, L and L', if present, each independently is a linker selected from an amino acid residue, a peptide moiety consisting of 2-6 amino acid residues, (C₁-C₈)alkylene, (C₂-C₈)alkenylene or (C₂-C₈)alkynylene, wherein said amino acid each independently is 6-aminohexanoic acid, 8-aminooctanoic acid, 1Nal, 2Nal, or Amc; and said (C₁-C₈)alkylene, (C₂-C₈)alkenylene and (C₂-C₈)alkynylene is optionally substituted with one or more groups each independently is selected from halogen, -COR₃, -COOR₃, -OCOOR₃, -OCON(R₃)₂, -CN, -NO₂, -SR₃, -OR₃, -N(R₃)₂, -CON(R₃)₂, -SO₂R₃, -SO₃H, or -S(=O)R₃, wherein R₃ is H, and further optionally interrupted by one or more identical or different heteroatoms selected from S, O or N, and/or at least one group selected from -NH-CO-, -CO-NH-, -N(C₁-C₈alkyl)-. More particular such embodiments are those wherein L and L' each independently is 6-aminohexanoic acid, 8-aminooctanoic acid, 1Nal-Amc, 2Nal-Amc, Amc-1Nal, or Amc-2Nal.

In certain embodiments, the PSMA ligand being labeled according to the method of the present invention is a compound of the general formula I, wherein R₁ is a glutamic acid residue; R₂ is a glutamic acid residue or a lysine residue preferably linked via its α-amino group to the adjacent -CO- group; L is 6-aminohexanoic acid, 8-aminooctanoic acid, 1Nal-Amc, 2Nal-Amc, Amc-1Nal, or Amc-2Nal; said radioactive isotope is ⁶⁸Ga, ¹⁷⁷Lu, or ⁹⁰Y; said chelating agent is HBED-CC or DOTA, forming amide bond with L; and X is absent. In one specific such embodiment, the PSMA ligand is a compound of the general formula I, wherein R₂ is a lysine residue linked via its α-amino group to the adjacent -CO- group; L is 6-aminohexanoic acid; and B is HBED-CC linked via a carboxylic group thereof to the amino group of the 6-aminohexanoic acid, i.e., 4,6,12,19-tetraazadocosane-1,3,7-tricarboxylic acid, 22-[3-[[[2-[[[5-(2-carboxyethyl)-2-hydroxyphenyl]methyl](carboxy-methyl)amino]ethyl] (carboxymethyl) amino]methyl]-4-hydroxyphenyl]-5,13,20-trioxo-(3S,7S)-trifluoroacetate salt, herein identified as **DKFZ-PSMA-11** (see Table 1). In other specific such embodiments, the PSMA ligand is a compound of the general formula I, wherein R₂ is a lysine residue linked via its α-amino group to the adjacent -CO- group; L is Amc-2Nal or Amc-1Nal linked via the carboxylic group of the 2Nal or 1Nal, respectively, to the side chain amino group of R₂; and B is DOTA linked via a carboxylic group thereof to the amino group of the Amc, i.e., 2-[3-(1-carboxy-5-{3-naphthalen-2-yl-2-[(4-{[2-(4,7,10-tris(carboxymethyl)-1,4,7,10-tetraaza-cyclododec-1-yl)-acetylamino]-methyl}-cyclohexanecarbonyl)-amino]-propionylamino}-pentyl)-ureido]-pentanedioic acid, herein identified as **DKFZ-PSMA-617**, or 2[-3-(1-carboxy-5-{3-naphthalen-1-yl-2-[(-4-{[2-(4,7,10-tris(carboxymethyl)-1,4,7,10-tetraaza-cyclododec-1-yl)-acetylamino]-methyl}-cyclohexanecarbonyl)-amino]-propionylamino}-pentyl)-ureido]-pentanedioic acid, herein identified as **DKFZ-PSMA-617-1** (see Table 1). According to the method of the invention, these specific PSMA ligands can be labeled with ⁶⁸Ga, ¹⁷⁷Lu, or ⁹⁰Y.

In other embodiments, the PSMA ligand being labeled according to the method of the present invention is a compound of the general formula I, wherein X is present; R₁ and R₁' are a glutamic acid residue; R₂ and R₂' are a glutamic acid residue or a lysine residue preferably linked via its α-amino group to the adjacent -CO- group; L and L' each independently is 6-aminohexanoic acid, 8-aminooctanoic acid, 1Nal-Amc, 2Nal-Amc, Amc-1Nal, or Amc-2Nal; said radioactive isotope is ⁶⁸Ga, ¹⁷⁷Lu, or ⁹⁰Y; and said chelating agent is HBED-CC or DOTA, forming amide bond with L, and another amide bond with L'. In one specific such embodiment, the PSMA ligand is a compound of the general formula I, wherein R₂ and R₂' are a lysine residue linked via its α-amino group to the adjacent -CO- group; L and L' each is 6-aminohexanoic acid; and B is HBED-CC linked via one carboxylic group thereof to the amino group of L and via another carboxylic group thereof to the amino group of L', i.e., (3S,7S)-22-(3-(((carboxymethyl)(2-((carboxymethyl)(2-hydroxy-5-((3R,7S)-1,3,7-tricarboxy-5,13,20-trioxo-4,6,12,19-tetraazadocosan-22-yl)benzyl)amino)ethyl)amino)methyl)-4-hydroxyphenyl)-5,13,20-trioxo-4,6,12,19-tetraazadocosane-1,3,7-tricarboxylic acid, herein identified as **DKFZ-PSMA-10** (see Table 1). According to the method of the invention, these specific PSMA ligands can be labeled with ⁶⁸Ga, ¹⁷⁷Lu, or ⁹⁰Y.

In certain embodiments, the present invention provides a method for labeling a PSMA ligand of the general formula I as defined in any one of the embodiments above, wherein the reaction vial provided in step (i) is pretreated with HCl, thereby adhering protons to the vial's wall. Such pretreatment is aimed at significantly reducing adherence of additional protons to the vial's wall upon introducing an eluted isotope's solution, and thus preventing or minimizing possible pH alterations of said solution and facilitating pH conditions favorable for the speedy complexation of the radioactive isotopes with the chelating agent, even at ambient temperature.

In certain embodiments, the present invention provides a method for labeling a PSMA ligand of the general formula I as defined in any one of the embodiments above, wherein said buffering agent in step (i) is any suitable sodium-based buffering agent such as sodium formate, sodium ascorbate, sodium acetate, sodium hydroxide, or sodium citrate. Specific embodiments are those wherein said buffering agent is sodium formate.

In certain embodiments, the present invention provides a method for labeling a PSMA ligand of the general formula I as defined in any one of the embodiments above, wherein the solution added in step (ii) to said reaction vial is obtained by eluting said radioactive isotope from a radioactive isotope generator, immediately before step (ii), using said predetermined amount of HCl as an elution solvent. Such embodiments are preferred, e.g., when the radioactive isotope used has a relatively short half-life such in the case of ⁶⁸Ga, which should thus be eluted from, e.g., a ⁶⁸Ge/⁶⁸Ga-generator, using said predetermined amount of HCl having a predetermined molarity, immediately before its addition to the reaction vial and mixing with said PSMA ligand and said sodium-based buffering agent.

According to the method of the present invention, the HCl solution obtained in step (ii), containing the PSMA ligand and the radioactive isotope, can be mixed using any suitable technique, e.g., by stirring or shaking, and is then incubated for a sufficient period of time and at a suitable temperature so as to react said PSMA ligand with said radioactive isotope, i.e., labeling said PSMA ligand with said radioactive isotope. According to the method of the invention, following this incubation step, at least 90%, 91%, 92%, 93%, 94%, 95%, 96%, 97%, 98%, or 99% of said radioactive isotope is bound to said PSMA ligand, i.e., coordinated with said chelating agent B. For example, in certain embodiments, the radioactive isotope is ⁶⁸Ga, and the solution in step (iii) is incubated for 2 to 10, 2 to 8, or 2 to 6, minutes at room temperature, so as to obtain at least 90% of said ⁶⁸Ga coordinated with said chelating agent B; or the radioactive isotope is ¹⁷⁷Lu or ⁹⁰Y, and the solution in step (iii) is incubated for about 30 minutes or more, e.g., for about 30, 35, 40, 45, or 50 minutes, at a temperature of about 100°C or higher, e.g., 100-110°C, 110-120°C, or 120-130°C, so as to obtain at least 95% or at least 99% of said ¹⁷⁷Lu or ⁹⁰Y coordinated with said chelating agent B.

Depending on the molarity of the HCl solution containing said radioactive isotope, and consequently the pH of the solution added to the reaction vial in step (ii), the pH of the labeled PSMA ligand-containing solution obtained in step (iv) might have to be adjusted to a pH compatible with physiological conditions, i.e., to a pH in a range of 4.0-8.0. The pH adjustment, if required, may be conducted by adding any suitable sodium-based buffering agent such as sodium formate, sodium ascorbate, sodium acetate, sodium hydroxide, or sodium citrate.

The Example section hereinafter shows preparation of ⁶⁸Ga-labeled **DKFZ-PSMA-11** in a disposable reaction vial containing particular amounts of **DKFZ-PSMA-11** and sodium formate, both in lyophilized form, by adding to said reaction vial ⁶⁸Ga eluted in a particular volume of HCl 0.1N, and incubating the resulting mixture at room temperature for about 5 minutes. The method exemplified is highly efficient, wherein following the short incubation of the PSMA ligand and the radioactive isotope, about 95% of the radioactive isotope is bound to said PSMA ligand, i.e., coordinated with the HBED-CC.

In one particular such aspect, the invention thus provides a method for labeling a PSMA ligand with ⁶⁸Ga, said method comprising the steps of: (i) providing a reaction vial containing about 800-1800 mg sodium formate and about 10 µg of said PSMA ligand, wherein both the sodium formate and said PSMA ligand are dried; (ii) adding a solution of ⁶⁸Ga in about 1.8 ml of HCl 0.1N to said reaction vial, thus obtaining a solution of said PSMA ligand and said ⁶⁸Ga in said HCl, wherein said solution of ⁶⁸Ga in HCl is obtained from a ⁶⁸Ga generator, immediately before this step, using said HCl as an elution solvent; (iii) mixing the solution obtained in (ii) and then incubating it for 2 to 10, preferably 2 to 6, minutes at room temperature, thus reacting said PSMA ligand with said ⁶⁸Ga and obtaining said PSMA ligand labeled with ⁶⁸Ga; and (iv) optionally adjusting the pH of the solution in said reaction vial to a pH in a range of 4.0 to 8.0, by adding about 0.1 ml sodium hydroxide 1N, wherein said PSMA ligand is of the general formula I':

**I'** R₁-CO-R₂-L-B

wherein R₁ is a glutamic acid residue linked via its amino group to the adjacent -CO-group; R₂ is a lysine residue linked via its α-amino group to the adjacent -CO- group; and (a) L is 6-aminohexanoic acid linked via the carboxylic group thereof to the to the side chain amino group of R₂; and B is HBED-CC linked via a carboxylic group thereof to the amino group of the 6-aminohexanoic acid; or (b) L is Amc-2Nal or Amc-1Nal linked via the carboxylic group of the 2Nal or 1Nal, respectively, to the side chain amino group of R₂; and B is DOTA linked via a carboxylic group thereof to the amino group of the Amc.

In another particular such aspect, the invention provides a method for labeling a PSMA ligand with ¹⁷⁷Lu or ⁹⁰Y, said method comprising the steps of: (i) providing a reaction vial containing sodium formate and 10 µg of said PSMA ligand, wherein both the sodium formate and said PSMA ligand are dried; (ii) adding a solution of ¹⁷⁷Lu or ⁹⁰Y in about 0.5 ml of HCl 0.1N to said reaction vial, thus obtaining a solution of said PSMA ligand and said ¹⁷⁷Lu or ⁹⁰Y in said HCl; and (iii) mixing the solution obtained in (ii) and then incubating it for about 30 minutes at a temperature of about 100°C or higher, thus reacting said PSMA ligand with said ¹⁷⁷Lu or ⁹⁰Y and obtaining said PSMA ligand labeled with ¹⁷⁷Lu or ⁹⁰Y, wherein said PSMA ligand is of the general formula I':

**I'** R₁-CO-R₂-L-B

wherein R₁ is a glutamic acid residue linked via its amino group to the adjacent -CO-group; R₂ is a lysine residue linked via its α-amino group to the adjacent -CO- group; L is Amc-2Nal or Amc-1Nal linked via the carboxylic group of the 2Nal or 1Nal, respectively, to the side chain amino group of R₂; and B is DOTA linked via a carboxylic group thereof to the amino group of the Amc.

Although in both particular aspects described above, 10 µg PSMA ligand were used, it should be noted that the amount of PSMA ligand may be modified, more particularly increased, so as to reduce reaction time. For instance, using 30 µg of PSMA ligand instead of 10 µg reduces the reaction time by up to 5 minutes when conducted at room temperature (for labeling said PSMA ligand with ⁶⁸Ga), and by up to 15-30 minutes when carried out at a temperature of about 100°C (for labeling said PSMA ligand with ¹⁷⁷Lu or ⁹⁰Y).

The radioactive isotope-labeled PSMA ligand prepared according to the method of the present invention can be administered to a subject, i.e., a mammal in general or a human in particular, for either imaging or treating a prostate cancer or metastases thereof, depending on the particular radioactive isotope used. In certain embodiments, the method of the invention in any one of the embodiments defined above further comprises a step of administering the solution obtained in step (iv) to a subject for imaging or for treatment of prostate cancer or metastases thereof.

A PSMA ligand labeled with a radioactive isotope by the method of the invention as defined in any one of the embodiments above, wherein said radioactive isotope is ⁶⁸Ga, may be used in imaging of prostate cancer or metastases thereof; and a PSMA ligand labeled by said method, wherein said radioactive isotope is ¹⁷⁷Lu or ⁹⁰Y, may be used in treatment of prostate cancer or metastases thereof.

A kit for carrying out the method of the invention is also disclosed, i.e., a kit comprising: (i) a disposable reaction vial containing a predetermined amounts of both a sodium-based buffering agent and a PSMA ligand of the general formula I as defined above, both in dried form, i.e., either lyophilized or spray dried; or two disposable reaction vials, wherein one of said reaction vials contains said sodium-based buffering agent and the other of said reaction vials contains said PSMA ligand, both in dried form; and (ii) instructions for optionally adding said sodium-based buffering agent to the reaction vial containing said PSMA ligand, and for labeling said PSMA ligand with a radioactive isotope. Sodium-based buffering agents that can be used in the kit include any suitable such buffering agents, e.g., sodium formate, sodium ascorbate, sodium acetate, sodium hydroxide, or sodium citrate.

The disposable reaction vial contained within the kit may be pretreated with HCl so as to adhere protons to the vial's wall. Such pretreatment is aimed at significantly reducing adherence of additional protons to the vial's wall upon introducing an eluted isotope's solution, and thus preventing or minimizing any pH alterations of said solution and facilitating pH conditions favorable for complexation of the radioactive isotopes with the chelating agent, even at ambient temperature.

The radioactive isotope for labeling the PSMA ligand provided with the kit may be provided as ready-for-use product, i.e., for mixing and incubating with the PSMA ligand and sodium-based buffering agent comprised within the kit, or alternatively may be eluted from a radioactive isotope generator prior to, and shortly before, mixing and incubating with said PSMA ligand and said sodium-based buffering agent, particularly in cases said radioactive isotope has a relatively short half-life such as in the case of ⁶⁸Ga. The kit defined above may thus further comprise at least one of: (i) a vial containing a predetermined amount of HCl having a molarity in a range of 0.05 to 0.1N for eluting said radioactive isotope from a radioactive isotope generator; and (ii) a vial containing a sodium-based buffering agent for pH adjustment. The disclosed kit does not form part of the invention.

The invention will now be illustrated by the following examples.

### EXAMPLES

### Example 1: Radio-synthesis of ⁶⁸Ga-labeled PSMA tracer according to a known technique

Disposable cassette kits and chemicals including the PSMA ligand **DKFZ-PSMA-11** in GMP-compliant grade used for the radio-synthesis were obtained from ABX advanced biochemical compounds (Germany).

The synthesis of the ⁶⁸Ga-labelled PSMA ligand as previously described (Eder M. et al., Novel preclinical and radiopharmaceutical aspects of [68Ga]Ga-PSMA-HBED-CC: A new PET tracer for imaging of prostate cancer, Pharmaceuticals, 2014, 7, 779-796) was reproduced reliably and slightly modified on a fully automated synthesis module in 80%±5% decay corrected radiochemical yield within 35 minutes, applying single-use cassette-based kits.

An audit trial was recorded for each radio-synthesis including all performed steps and courses of radioactivity on three gamma detectors, gas flow and temperature of the heating unit. The steps involved include: (i) conditioning of the purification cartridge; (ii) elution of the ⁶⁸Ge/⁶⁸Ga-generator; (iii) purification of the generator elute; (iv) radio-synthesis of the radiolabeled PSMA ligand; and (v) purification by cation exchange column filtration and sterile filtration of the final product.

The ⁶⁸Ga-labelled **DKFZ-PSMA-11** was obtained in >95% radiochemical purity as two diastereomers with a pH ranging between 6 and 8. Stability of this tracer in the final product solution was tested for up to 6 hours after the end of radio-synthesis and yielded identical results for the radiochemical purity. The only residual solvent found in the final product solution was ethanol in less than 5% v/v taking the density at 20°C to be 0.79 g/ml. Bacterial endotoxin testing showed <2 IU/ml and all samples tested were sterile. Filter integrity of the sterile filter was tested using the bubble-point test. Gamma spectrometry showed characteristic peaks at 0.511 and 1.077 MeV.

### Example 2: Two modes of operation for using the automated radio-synthesis module (not forming part of the invention)

The automated synthesis module can be carried out using either reusable or disposable reaction vials.

When reusable vials are used, the mode of operation includes the steps of: (a) adding into the reaction vial a PSMA ligand and sodium formate to reach a working pH of from about 4.0 to about 8.0; (b) automatically eluting the radioactive isotope generator by fractionation with HCl 0.1N (about 0.5 ml for ⁹⁰Y or ¹⁷⁷Lu, or about 1.8 ml for ⁶⁸Ga); (c) heating the reaction vial at about 95°C (30 minutes for ⁹⁰Y or ¹⁷⁷Lu, or 5 minutes for ⁶⁸Ga); (d) purifying the radiolabeled PSMA tracer by passing the crude product through cation exchange column, where the labeled PSMA tracer remains on the column and the free radioactive isotopes are removed and discarded; (e) passing saline through said column to remove residual HCl; (f) eluting the labeled PSMA tracer from the cation exchange column with about 0.5 ml 50% ethanol; (g) passing saline through said column to dilute the final product to a max 5% ethanol; and (h) sterilizing the elute by passing it through a 0.22 micron filter into a collection vial. Finally, a quality control of the labeled PSMA tracer is performed.

When disposable vials and reagents are used, in which case all the reagents are contained in a disposable vial and disposable separation-column, the mode of operation includes the same steps mentioned above, with the exeption that the vials and the cation exchange column being used are disposable and therefore do not need to be washed after use.

### Example 3: Radio-synthesis of ⁶⁸Ga-labelled DKFZ-PSMA-11 using the "shake & bake" method

Radio-synthesis of ⁶⁸Ga-labeled DKFZ-PSMA-11 was carried out using a kit comprising (i) a disposable reaction vial containing the PSMA ligand (10 µg) and sodium formate (800-1800 mg), both in lyophilized form; (ii) an elution vial containing 0.1N HCl, for elution of the ⁶⁸Ge/⁶⁸Ga generator; and (iii) a buffer vial containing 1N NaOH, for pH adjustment.

The procedure of radiolabeling the PSMA tracer was performed by: (a) eluting the ⁶⁸Ge/⁶⁸Ga generator by fractionation with about 1.8 ml HCl 0.1N directly into the reaction vial; (b) mixing and incubating the reaction vial at room temperature for about 5 minutes; and (c) adjusting the pH in the reaction vial, if lower than 4.0, by adding 0.1 ml 1N NaOH until reaching a pH in the range of 4.0 to 8.0.

A similar experiment conducted with a disposable reaction vial containing 30 µg of the PSMA ligand, reduced the incubation time by about 2 to 3 minutes.

### Example 4: Quality control procedures

The resulting ⁶⁸Ga-PSMA tracers produced in Examples 1 and 3 above were tested for radiochemical purity by using thin layer chromatography (TLC). The resulting acceptable criteria using 0.1M sodium citrate as the runing solvent are: (a) for free ⁶⁸Ga: Retardation Factor (RF) of 0.8-1.0 and no more than 10%; and (b) for ⁶⁸Ga-labeled tracer: RF of 0.0-0.3 and no less than 90%. The resulting acceptable criteria using 1M ammonium acetate+methanol (1:1) as the running solvent are (a) for colloid ⁶⁸Ga: RF of 0.0-0.2 and no more than 10%; and (b) for ⁶⁸Ga labeled tracer: RF of 0.8-1.0 and no less than 90%.

The half-life (t_{½}) of the resulting ⁶⁸Ga-PSMA tracer was from about 65.0 to about 69.6 minutes.

**Table 1**

| **Specific PSMA ligands described herein** | |
|---|---|
| **DKFZ-PSMA-11** | 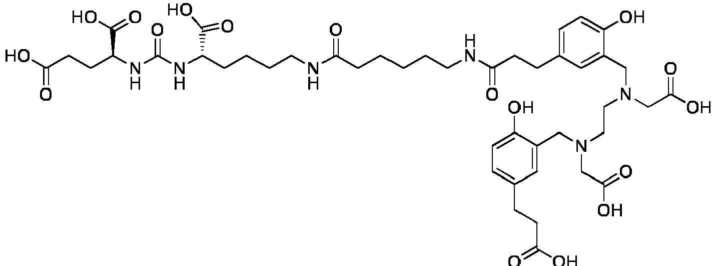 |
| **DKFZ-PSMA-617** | 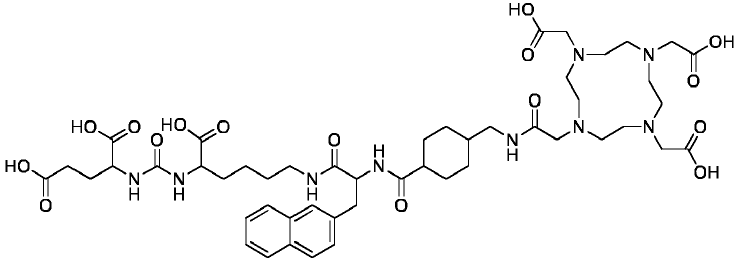 |
| **DKFZ-PSMA-617-1** | 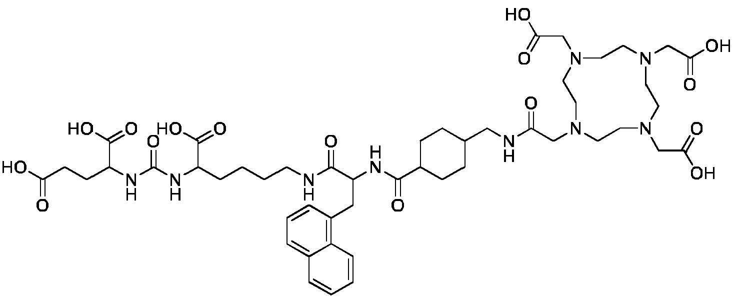 |
| **DKFZ-PSMA-10** | 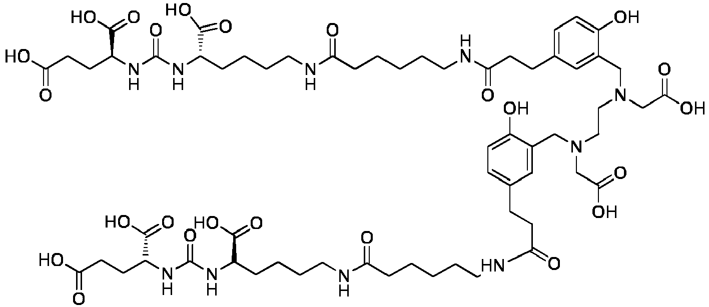 |

## Claims

1. A method for labeling a prostate-specific membrane antigen (PSMA) ligand with a radioactive isotope selected from ⁶⁸Ga, ¹⁷⁷Lu, or ⁹⁰Y, said method comprising the steps of:
(i) providing a reaction vial containing a predetermined amounts of a sodium-based buffering agent and said PSMA ligand, both in dried form;
(ii) adding a solution of said radioactive isotope in a predetermined amount of HCl having a predetermined molarity to said reaction vial, thus obtaining a solution of said PSMA ligand and said radioactive isotope in said HCl;
(iii) mixing the solution obtained in (ii) and then incubating it for a sufficient period of time, thus reacting said PSMA ligand with said radioactive isotope to thereby obtain said PSMA ligand labeled with said radioactive isotope, wherein at least 90% of said radioactive isotope in said solution is bound to said PSMA ligand; and
(iv) optionally adjusting the pH of the solution in said reaction vial to a pH compatible with physiological conditions by adding a sodium-based buffering agent,
wherein said PSMA ligand is of the general formula I:
**I** R₁-CO-R₂-L-B-X
wherein
B is a chelating agent capable of coordinating with said radioactive isotope;
X is absent or is of the formula -L'-R₂'-CO- R₁';
L and L' each independently is absent or a linker;
R₁ and R₁', if present, each is a glutamic acid residue linked via the amino group thereof to the adjacent -CO- group; and
R₂ and R₂', if present, each is a glutamic acid residue or a lysine residue linked via an amino group thereof to the adjacent -CO- group.

2. The method of claim 1, wherein:
(i) L and L' each independently is absent or a linker selected from an amino acid residue forming a peptide bond with R₂ or R₂', respectively, a peptide moiety consisting of 2-6 amino acid residues and forming a peptide bond with R₂ or R₂', respectively, (C₁-C₈)alkylene, (C₂-C₈)alkenylene or (C₂-C₈)alkynylene, wherein said (C₁-C₈)alkylene, (C₂-C₈)alkenylene and (C₂-C₈)alkynylene is optionally substituted with one or more groups each independently is selected from halogen, -COR₃, -COOR₃, -OCOOR₃, - OCON(R₃)₂, -CN, -NO₂, -SR₃, -OR₃, -N(R₃)₂, -CON(R₃)₂, -SO₂R₃, -SO₃H, or -S(=O)R₃, and further optionally interrupted by one or more identical or different heteroatoms selected from S, O or N, and/or at least one group selected from -NH-CO-, -CO-NH-, or -N(C₁-C₈alkyl)-, wherein R₃ each independently is selected from hydrogen, or -(C₁-C₈)alkyl; or
(ii) said chelating agent is N,N'-bis[2-hydroxy-5-(carboxyethyl)benzyl] ethylenediamine-N,N'-diacetic acid (HBED-CC) or 1,4,7,10-tetraazacyclododecane-1,4,7,10-tetraacetic acid (DOTA), forming amide bond with either L, if present, or R₂, and when X is present, forming another amide bond with either L', if present, or R₂'.

3. The method of claim 2, wherein:
(i) said linker is an amino acid residue, or a peptide moiety consisting of 2-6 amino acid residues, wherein said amino acid each independently is 6-aminohexanoic acid, 8-aminooctanoic acid, 1-naphthylalanine (1Nal), 2-naphthylalanine (2Nal), or 4-(aminomethyl)cyclohexane carboxylic acid (Amc), preferably a residue of 6-aminohexanoic acid or 8-aminooctanoic acid, or a moiety of 1Nal-Amc, 2Nal-Amc, Amc-1Nal or Amc-2Nal; or
(ii) said linker each independently is (C₁-C₈)alkylene, (C₂-C₈)alkenylene or (C₂-C₈)alkynylene, optionally substituted with one or more groups each independently is selected from halogen, -COH, -COOH, -OCOOH, - OCONH₂, -CN, -NO₂, -SH, -OH, -NH₂, -CONH₂, -SO₂H, -SO₃H, or - S(=O)H, and further optionally interrupted by one or more identical or different heteroatoms selected from S, O or N, and/or at least one group selected from -NH-CO-, -CO-NH-, or -N(C₁-C₈alkyl)-.

4. The method of claim 1, wherein:
L and L' each independently is absent or a linker selected from an amino acid residue forming a peptide bond with R₂ or R₂', respectively, a peptide moiety consisting of 2-6 amino acid residues and forming a peptide bond with R₂ or R₂', respectively, (C₁-Cs)alkylene, (C₂-C₈)alkenylene or (C₂-C₈)alkynylene, wherein said (C₁-C₈)alkylene, (C₂-C₈)alkenylene and (C₂-C₈)alkynylene each independently is optionally substituted with one or more groups each independently is selected from halogen, -COR₃, -COOR₃, -OCOOR₃, -OCON(R₃)₂, -CN, -NO₂, -SR₃, -OR₃, -N(R₃)₂, -CON(R₃)₂, -SO₂R₃, -SO₃H, or -S(=O)R₃, and further optionally interrupted by one or more identical or different heteroatoms selected from S, O or N, and/or at least one group selected from -NH-CO-, -CO-NH-, or -N(C₁-C₈alkyl)-, wherein R₃ each independently is selected from hydrogen, or -(C₁-C₈)alkyl; and
said chelating agent is HBED-CC or DOTA, forming an amide bond with either L, if present, or R₂, and when X is present, forming another amide bond with either L', if present, or R₂'.

5. The method of claim 4, wherein L and L', if present, each independently is a linker selected from an amino acid residue, a peptide moiety consisting of 2-6 amino acid residues, (C₁-C₈)alkylene, (C₂-C₈)alkenylene or (C₂-C₈)alkynylene, wherein said amino acid each independently is 6-aminohexanoic acid, 8-aminooctanoic acid, 1Nal, 2Nal, or Amc; and said (C₁-C₈)alkylene, (C₂-C₈)alkenylene and (C₂-C₈)alkynylene is optionally substituted with one or more groups each independently is selected from halogen, -COH, -COOH, -OCOOH, -OCONH₂, -CN, -NO₂, -SH, -OH, -NH₂, -CONH₂, -SO₂H, -SO₃H, or -S(=O)H, and further optionally interrupted by one or more identical or different heteroatoms selected from S, O or N, and/or at least one group selected from -NH-CO-, -CO-NH-, or -N(C₁-C₈alkyl)-.

6. The method of claim 5, wherein L and L' each independently is a residue of 6-aminohexanoic acid or 8-aminooctanoic acid, or a moiety of 1Nal-Amc, 2Nal-Amc, Amc-1Nal or Amc-2Nal.

7. The method of claim 6, wherein (i) X is absent; R₂ is a lysine residue linked via its α-aminogroup to the adjacent -CO- group; and (a) L is 6-aminohexanoic acid; and B is HBED-CC linked via a carboxylic group thereof to the amino group of the 6-aminohexanoic acid; or (b) L is Amc-2Nal or Amc-1Nal linked via the carboxylic group of the 2Nal or 1Nal, respectively, to the side chain amino group of R₂; and B is DOTA linked via a carboxylic group thereof to the amino group of the Amc; or (ii) X is present; R₂ and R₂' each is a lysine residue linked via its α-amino group to the adjacent - CO- group; L and L' each is 6-aminohexanoic acid; and B is HBED-CC linked via one carboxylic group thereof to the amino group of L and via another carboxylic group thereof to the amino group of L'.

8. The method of claim 7, wherein said radioactive isotope is ⁶⁸Ga.

9. The method of any one of claim 1 to 8, wherein
(a) said reaction vial provided in step (i) is pretreated with HCl; or
(b) said buffering agent in step (i) is sodium formate, sodium ascorbate, sodium acetate, sodium hydroxide, or sodium citrate; or
(c) the solution added in step (ii) to said reaction vial is obtained by eluting said radioactive isotope from a radioactive isotope generator, immediately before step (ii), using said predetermined amount of HCl as an elution solvent; or
(d) said mixing in step (iii) is carried out by stirring or shaking; or
(e) said radioactive isotope is ⁶⁸Ga, and the solution in step (iii) is incubated for 2 to 10, preferably 2 to 6, minutes at room temperature; or said radioactive isotope is ¹⁷⁷Lu or ⁹⁰Y, and the solution in step (iii) is incubated for about 30 minutes or more at a temperature of about 100°C or higher; or
(f) the pH of the solution in step (iv) is adjusted to a pH in a range of 4.0 to 8.0, by adding sodium formate, sodium ascorbate, sodium acetate, sodium hydroxide, or sodium citrate.

10. A method for labeling a PSMA ligand with ⁶⁸Ga, said method comprising the steps of:
(i) providing a reaction vial containing about 800-1800 mg sodium formate and about 10 µg of said PSMA ligand, wherein both the sodium formate and said PSMA ligand are dried;
(ii) adding a solution of ⁶⁸Ga in about 1.8 ml of HCl 0.1N to said reaction vial, thus obtaining a solution of said PSMA ligand and said ⁶⁸Ga in said HCl, wherein said solution of ⁶⁸Ga in HCl is obtained from a ⁶⁸Ga generator, immediately before this step, using said HCl as an elution solvent;
(iii) mixing the solution obtained in (ii) and then incubating it for 2 to 10, preferably 2 to 6, minutes at room temperature, thus reacting said PSMA ligand with said ⁶⁸Ga and obtaining said PSMA ligand labeled with ⁶⁸Ga; and
(iv) optionally adjusting the pH of the solution in said reaction vial to a pH in a range of 4.0 to 8.0, by adding about 0.1 ml sodium hydroxide 1N,
wherein said PSMA ligand is of the general formula I':
**I'** R₁-CO-R₂-L-B
wherein
R₁ is a glutamic acid residue linked via its amino group to the adjacent -CO-group; R₂ is a lysine residue linked via its α-amino group to the adjacent -CO- group; and either: (a) L is 6-aminohexanoic acid linked via the carboxylic group thereof to the side chain amino group of R₂; and B is HBED-CC linked via a carboxylic group thereof to the amino group of the 6-aminohexanoic acid; or (b) L is Amc-2Nal or Amc-1Nal linked via the carboxylic group of the 2Nal or 1Nal, respectively, to the side chain amino group of R₂; and B is DOTA linked via a carboxylic group thereof to the amino group of the Amc.

11. A method for labeling a PSMA ligand with ¹⁷⁷Lu or ⁹⁰Y, said method comprising the steps of:
(i) providing a reaction vial containing sodium formate and 10 µg of said PSMA ligand, wherein both the sodium formate and said PSMA ligand are dried;
(ii) adding a solution of ¹⁷⁷Lu or ⁹⁰Y in about 0.5 ml of HCl 0.1N to said reaction vial, thus obtaining a solution of said PSMA ligand and said ¹⁷⁷Lu or ⁹⁰Y in said HCl; and
(iii) mixing the solution obtained in (ii) and then incubating it for about 30 minutes at a temperature of about 100°C or higher, thus reacting said PSMA ligand with said ¹⁷⁷Lu or ⁹⁰Y and obtaining said PSMA ligand labeled with ¹⁷⁷Lu or ⁹⁰Y,
wherein said PSMA ligand is of the general formula I':
**I'** R₁-CO-R₂-L-B
wherein
R₁ is a glutamic acid residue linked via its amino group to the adjacent -CO-group; R₂ is a lysine residue linked via its α-amino group to the adjacent -CO- group; and L is Amc-2Nal or Amc-1Nal linked via the carboxylic group of the 2Nal or 1Nal, respectively, to the side chain amino group of R₂; and B is DOTA linked via a carboxylic group thereof to the amino group of the Amc.

## Patentansprüche

1. Verfahren zur Markierung eines prostataspezifischen Membranantigen (PSMA)-Liganden mit einem radioaktiven Isotop, ausgewählt aus ⁶⁸Ga, ¹⁷⁷Lu oder ⁹⁰Y, wobei das Verfahren die folgenden Schritte umfasst:
(i) Bereitstellen eines Reaktionsfläschchens, das eine vorbestimmte Menge eines natriumbasierten Puffermittels und des PSMA-Liganden enthält, beide in getrockneter Form;
(ii) Zugabe einer Lösung des radioaktiven Isotops in einer vorbestimmten Menge HCl mit einer vorbestimmten Molarität zu dem Reaktionsfläschchen, wodurch eine Lösung des PSMA-Liganden und des radioaktiven Isotops in dem HCl erhalten wird;
(iii) Mischen der in (ii) erhaltenen Lösung und anschließendes Inkubieren für einen ausreichenden Zeitraum, wodurch der PSMA-Ligand mit dem radioaktiven Isotop umgesetzt wird, um dadurch den mit dem radioaktiven Isotop markierten PSMA-Liganden zu erhalten, wobei mindestens 90 % des radioaktiven Isotops in der Lösung an den PSMA-Liganden gebunden sind; und
(iv) optional Einstellen des pH-Werts der Lösung in dem Reaktionsfläschchen auf einen pH-Wert, der mit physiologischen Bedingungen kompatibel ist, durch Zugabe eines natriumbasierten Puffermittels,
wobei der PSMA-Ligand gemäß der allgemeinen Formel I definert ist:
I R₁-CO-R₂-L-B-X
wobei
B ist ein Chelatbildner, der in der Lage ist, sich mit dem radioaktiven Isotop zu koordinieren;
X ist nicht vorhanden oder entspricht der Formel -L'-R₂'-CO- R₁';
L und L' ist jeweils unabhängig nicht vorhanden oder ein Linker;
R₁ und R₁', falls vorhanden, ist jeweils ein Glutaminsäurerest, der über seine Aminogruppe mit der benachbarten -CO-Gruppe verknüpft ist; und
R₂ und R₂', falls vorhanden, sind jeweils ein Glutaminsäurerest oder ein Lysinrest, der über eine Aminogruppe davon mit der benachbarten -CO-Gruppe verknüpft ist.

2. Verfahren nach Anspruch 1, wobei:
(i) L und L' sind jeweils unabhängig voneinander nicht vorhanden oder ein Linker, ausgewählt aus einem Aminosäurerest, der eine Peptidbindung mit R₂ bzw. R₂' bildet, eine Peptideinheit, die aus 2-6 Aminosäureresten besteht und eine Peptidbindung mit R₂ bzw. R₂' bildet, (C₁-C₈)Alkylen, (C₂-C₈)Alkenylen oder (C₂-C₈)Alkinylen, wobei das (C₁-C₈)Alkylen, (C₂-C₈)Alkenylen und (C₂-C₈)Alkinylen gegebenenfalls mit einer oder mehreren Gruppen substituiert ist, die jeweils unabhängig voneinander aus Halogen -COR₃, -COOR₃, -OCOOR₃, -OCON(R₃)₂, -CN, -NO₂, -SR₃, - OR₃, -N(R₃)₂, -CON(R₃)₂, -SO₂R₃, -SO₃H oder -S(=O)R₃ ausgewählt ist, und ferner optional unterbrochen durch ein oder mehrere identische oder unterschiedliche Heteroatome, ausgewählt aus S, O oder N, und/oder mindestens eine Gruppe ausgewählt aus -NH-CO-, -CO-NH- oder -N(C₁-C₈alkyl)-, wobei R₃ jeweils unabhängig voneinander aus Wasserstoff oder - (C₁-C₈)Alkyl ausgewählt ist; oder
(ii) der Chelatbildner ist N,N'-Bis[2-hydroxy-5-(carboxyethyl)benzyl]ethylendiamin-N,N'-diessigsäure (HBED-CC) oder 1,4,7,10-tetraazacyclododecan-1,4,7,10-tetraessigsäure (DOTA), die eine Amidbindung entweder mit L, falls vorhanden, oder mit R₂ bildet, und, wenn X vorhanden ist, eine weitere Amidbindung mit L', falls vorhanden, oder R₂' bildet.

3. Verfahren nach Anspruch 2, wobei:
(i) der Linker ein Aminosäurerest oder eine Peptideinheit ist, die aus 2-6 Aminosäureresten besteht, wobei die Aminosäure unabhängig voneinander 6-Aminohexansäure, 8-Aminooctansäure, 1-Naphthylalanin (1Nal), 2-Naphthylalanin (2Nal) oder 4-(Aminomethyl)cyclohexancarbonsäure (Amc), vorzugsweise ein Rest von 6-Aminohexansäure oder 8-Aminooctansäure, oder ein Teil von 1Nal-Amc, 2Nal-Amc, Amc-1Nal oder Amc-2Nal ist; oder
(ii) wobei der Linker jeweils unabhängig voneinander (C₁-C₈)Alkylen, (C₂-C₈)Alkenylen oder (C₂-C₈)Alkinylen ist, optional mit einer oder mehreren Gruppen substituiert ist, die jeweils unabhängig voneinander aus Halogen, -COH, -COOH, -OCOOH, -OCONH₂, -CN, -NO₂, -SH, -OH, -NH₂, - CONH₂, -SO₂H, -SO₃H oder -S(=O)H ausgewählt ist, und ferner gegebenenfalls unterbrochen durch ein oder mehrere identische oder unterschiedliche Heteroatome, ausgewählt aus S, O oder N und/oder mindestens eine Gruppe, ausgewählt aus -NH-CO-, -CO-NH- oder -N(C₁-Csalkyl)-.

4. Verfahren nach Anspruch 1, wobei:
L und L' sind jeweils unabhängig voneinander nicht vorhanden oder ein Linker, ausgewählt aus einem Aminosäurerest, der eine Peptidbindung mit R₂ bzw. R₂' bildet, eine Peptideinheit, die aus 2-6 Aminosäureresten besteht und eine Peptidbindung mit R₂ bzw. R₂' bildet, (C₁-C₈)Alkylen, (C₂-C₈)Alkenylen oder (C₂-C₈)Alkinylen, wobei optional (C₁-C₈)Alkylen, (C₂-C₈)Alkenylen und (C₂-C₈)Alkinylen jeweils unabhängig voneinander mit einer oder mehreren Gruppen substituiert ist, jeweils unabhängig voneinander aus Halogen, -COR₃, -COOR₃, -OCOOR₃, -OCON(R₃)₂, -CN, -NO₂, -SR₃, -OR₃, -N(R₃)₂, -CON(R₃)₂, -SO₂R₃, -SO₃H oder -S(=O)R₃ ausgewählt ist, und ferner optional unterbrochen durch ein oder mehrere identische oder unterschiedliche Heteroatome, ausgewählt aus S, O oder N, und/oder mindestens eine Gruppe ausgewählt aus -NH-CO-, -CO-NH- oder -N(C₁-C₈)Alkyl-, wobei R₃ jeweils unabhängig voneinander aus Wasserstoff oder -(C₁-C₈)Alkyl ausgewählt ist; und
der Chelatbildner ist HBED-CC oder DOTA, bildet eine Amidbindung entweder mit L, falls vorhanden, oder mit R₂, und, wenn X vorhanden ist, bildet eine weitere Amidbindung mit L', falls vorhanden, oder R₂'.

5. Verfahren nach Anspruch 4, wobei L und L', falls vorhanden, jeweils unabhängig voneinander ein Linker ist, ausgewählt aus einem Aminosäurerest, einer Peptideinheit, die aus 2-6 Aminosäureresten besteht, (C₁-C₈)Alkylen, (C₂-C₈)Alkenylen oder (C₂-C₈)Alkinylen, wobei die Aminosäure jeweils unabhängig voneinander 6-Aminohexansäure, 8-Aminooctansäure, 1Nal, 2Nal oder Amc ist; und das (C₁-C₈)Alkylen, (C₂-C₈)Alkenylen und (C₂-C₈)Alkinylen optional mit einer oder mehreren Gruppen substituiert ist, die jeweils unabhängig voneinander aus Halogen, -COH, -COOH, -OCOOH, -OCONH₂, -CN, -NO₂, -SH, -OH, -NH₂, -CONH₂, -SO₂H, -SO₃H oder -S(=O)H ausgewählt ist und ferner gegebenenfalls unterbrochen durch ein oder mehrere identische oder unterschiedliche Heteroatome, ausgewählt aus S, O oder N, und/oder mindestens eine Gruppe, ausgewählt aus -NH-CO-, -CO-NH- oder -N(C₁-C₈)Alkyl-.

6. Verfahren nach Anspruch 5, wobei L und L' unabhängig voneinander jeweils ein Rest von 6-Aminohexansäure oder 8-Aminooctansäure oder ein Teil von 1Nal-Amc, 2Nal-Amc, Amc-1Nal oder Amc-2Nal ist.

7. Verfahren nach Anspruch 6, wobei (i) X fehlt; R₂ ist ein Lysinrest, der über seine α-Aminogruppe mit der benachbarten -CO-Gruppe verknüpft ist; und (a) L für 6-Aminohexansäure steht; und B HBED-CC ist, das über eine Carbonsäuregruppe davon an die Aminogruppe der 6-Aminohexansäure gebunden ist; oder (b) L Amc-2Nal oder Amc-1Nal ist, die über die Carboxylgruppe des 2Nal bzw. 1Nal mit der Seitenkettenaminogruppe von R₂ verknüpft sind; und B DOTA ist, das über eine Carboxylgruppe davon mit der Aminogruppe des Amc verknüpft ist; oder (ii) X vorhanden ist; R₂ und R₂' sind jeweils ein Lysinrest, der über seine α-Aminogruppe mit der benachbarten -CO-Gruppe verknüpft ist; L und L' jeweils 6-Aminohexansäure sind; und B HBED-CC über eine Carbonsäuregruppe davon mit der Aminogruppe von L und über eine andere Carbonsäuregruppe davon mit der Aminogruppe von L' verknüpft ist.

8. Verfahren nach Anspruch 7, wobei das radioaktive Isotop ⁶⁸Ga ist.

9. Verfahren nach einem der Ansprüche 1 bis 8, wobei
(a) das in Schritt (i) vorgesehene Reaktionsfläschchen mit HCl vorbehandelt ist; oder
(b) das Puffermittel in Schritt (i) Natriumformiat, Natriumascorbat, Natriumacetat, Natriumhydroxid oder Natriumcitrat ist; oder
(c) die Lösung, die in Schritt (ii) zu dem Reaktionsfläschchen hinzugefügt wird, erhält man durch Elution des radioaktiven Isotops aus einem radioaktiven Isotopengenerator unmittelbar vor Schritt (ii), wobei die vorbestimmte Menge an HCl als Elutionslösungsmittel verwendet wird; oder
(d) wobei das Mischen in Schritt (iii) durch Rühren oder Schütteln erfolgt; oder
(e) das radioaktive Isotop ⁶⁸Ga ist, und die Lösung in Schritt (iii) wird für 2 bis 10, vorzugsweise 2 bis 6 Minuten bei Raumtemperatur inkubiert; oder das radioaktive Isotop ¹⁷⁷Lu oder ⁹⁰Y ist und die Lösung in Schritt (iii) etwa 30 Minuten oder länger bei einer Temperatur von etwa 100 °C oder höher inkubiert wird; oder
(f) der pH-Wert der Lösung in Schritt (iv) wird durch Zugabe von Natriumformiat, Natriumascorbat, Natriumacetat, Natriumhydroxid oder Natriumcitrat auf einen pH-Wert in einem Bereich von 4,0 bis 8,0 eingestellt.

10. Verfahren zur Markierung eines PSMA-Liganden mit ⁶⁸Ga, wobei das Verfahren die folgenden Schritte umfasst:
(i) Bereitstellen eines Reaktionsfläschchens, das etwa 800-1800 mg Natriumformiat und etwa 10 µg des PSMA-Liganden enthält, wobei sowohl das Natriumformiat als auch der PSMA-Ligand getrocknet sind;
(ii) Zugabe einer Lösung von ⁶⁸Ga in etwa 1,8 ml HCl 0,1N zu dem Reaktionsfläschchen, wodurch eine Lösung des PSMA-Liganden und des ⁶⁸Ga in dem HCl erhalten wird, wobei die Lösung von ⁶⁸Ga in HCl unmittelbar vor diesem Schritt aus einem ⁶⁸Ga-Generator erhalten wird, wobei das HCl als Elutionslösungsmittel verwendet wird;
(iii) Mischen der in (ii) erhaltenen Lösung und anschließendes Inkubieren für 2 bis 10, vorzugsweise 2 bis 6 Minuten bei Raumtemperatur, wodurch der PSMA-Ligand mit dem ⁶⁸Ga umgesetzt und der mit ⁶⁸Ga markierte PSMA-Ligand erhalten wird; und
(iv) optional Einstellen des pH-Wertes der Lösung in dem Reaktionsfläschchen auf einen pH-Wert in einem Bereich von 4,0 bis 8,0 durch Zugabe von etwa 0,1 ml Natriumhydroxid 1N,
wobei der PSMA-Ligand die allgemeine Formel I' hat:
**I'** R₁-CO-R₂-L-B
wobei
R₁ ein Glutaminsäurerest ist, der über seine Aminogruppe mit der benachbarten -CO-Gruppe verknüpft ist; R₂ ist ein Lysinrest, der über seine α-Aminogruppe mit der benachbarten -CO-Gruppe verknüpft ist; und entweder: (a) L für 6-Aminohexansäure steht, die über deren Carbonsäuregruppe mit der Seitenkettenaminogruppe von R₂ verknüpft ist; und B HBED-CC ist, das über eine Carbonsäuregruppe davon an die Aminogruppe der 6-Aminohexansäure gebunden ist; oder (b) L Amc-2Nal oder Amc-1Nal ist, die über die Carboxylgruppe des 2Nal bzw. 1Nal mit der Seitenkettenaminogruppe von R₂ verknüpft sind; und B steht für DOTA, das über eine Carboxylgruppe davon mit der Aminogruppe des Amc verknüpft ist.

11. Verfahren zur Markierung eines PSMA-Liganden mit ¹⁷⁷Lu oder ⁹⁰Y, wobei das Verfahren die folgenden Schritte umfasst:
(i) Bereitstellen eines Reaktionsfläschchens, das Natriumformiat und 10 µg des PSMA-Liganden enthält, wobei sowohl das Natriumformiat als auch der PSMA-Ligand getrocknet sind;
(ii) Zugabe einer Lösung von ¹⁷⁷Lu oder ⁹⁰Y in etwa 0,5 ml HCl 0,1N zu dem Reaktionsfläschchens, wodurch eine Lösung des PSMA-Liganden und der ¹⁷⁷Lu oder ⁹⁰Y in dem HCl erhalten wird; und
(iii) Mischen der in (ii) erhaltenen Lösung und anschließendes Inkubieren für etwa 30 Minuten bei einer Temperatur von etwa 100 °C oder höher, wodurch der PSMA-Ligand mit ¹⁷⁷Lu oder ⁹⁰Y umgesetzt wird und der PSMA-Ligand mit ¹⁷⁷Lu oder ⁹⁰Y markiert wird,
wobei der PSMA-Ligand gemäß der allgemeinen Formel I ist':
**I'** R₁-CO-R₂-L-B
wobei
R₁ ist ein Glutaminsäurerest, der über seine Aminogruppe mit der benachbarten -CO-Gruppe verknüpft ist; R₂ ist ein Lysinrest, der über seine α-Aminogruppe mit der benachbarten -CO-Gruppe verknüpft ist; und L für Amc-2Nal oder Amc-1Nal steht, die über die Carboxylgruppe des 2Nal bzw. 1Nal mit der Seitenkettenaminogruppe von R₂ verknüpft sind; und B steht für DOTA, das über eine Carboxylgruppe davon mit der Aminogruppe des Amc verknüpft ist.

## Revendications

1. Un procédé de marquage d'un ligand d'un antigène membranaire spécifique de la prostate (PSMA) avec un isotope radioactif choisi parmi ⁶⁸Ga, ¹⁷⁷Lu, ou ⁹⁰Y, ledit procédé comprenant les étapes consistant à :
(i) fournir un flacon de réaction contenant des quantités prédéterminées d'un agent tampon à base de sodium et dudit ligand PSMA, tous deux sous forme séchée ;
(ii) ajouter audit flacon de réaction une solution dudit isotope radioactif dans une quantité prédéterminée de HCl ayant une molarité prédéterminée, de façon à obtenir ainsi une solution dudit ligand PSMA et dudit isotope radioactif dans ledit HCl ;
(iii) mélanger la solution obtenue en (ii) puis l'incuber pendant une période de temps suffisante, faisant ainsi réagir ledit ligand PSMA avec ledit isotope radioactif pour obtenir ainsi ledit ligand PSMA marqué avec ledit isotope radioactif, au moins 90 % dudit isotope radioactif dans ladite solution étant lié audit ligand PSMA ; et
(iv) optionnellement ajuster le pH de la solution dans ledit flacon de réaction à un pH compatible avec les conditions physiologiques par ajout d'un agent tampon à base de sodium,
ledit ligand PSMA répondant à la formule générale I :
**I** R₁-CO-R₂-L-B-X
dans laquelle
B est un agent chélateur apte à se coordonner avec ledit isotope radioactif ;
X est absent ou est de formule -L'-R₂'-CO-R₁' :
L et L' sont chacun indépendamment absents ou constituent un lieur ("*linker*") ;
R₁ et R₁', s'ils sont présents, représentent chacun un résidu d'acide glutamique lié via son groupe amino au groupe -CO- adjacent ; et
R₂ et R₂', s'ils sont présents, représentent chacun un résidu d'acide glutamique ou un résidu lysine lié via un groupe amino de celui-ci au groupe -CO- adjacent.

2. Le procédé selon la revendication 1, dans lequel :
(i) L et L' sont chacun indépendamment absents ou un lieur sélectionné parmi un résidu d'acide aminé formant une liaison peptidique avec R₂ ou R₂', respectivement, un fragment peptidique constitué de 2 à 6 résidus d'acides aminés et formant une liaison peptidique avec R₂ ou R₂', respectivement, alkylène (C₁-C₈), alcénylène (C₂-C₈) ou alcynylène (C₂-C₈), ledit alkylène (C₁-C₈), alcénylène (C₂-C₈) et alcynylène (C₂-Cs) est optionnellement substitué par un ou plusieurs groupes, chacun étant choisi indépendamment parmi halogène, -COR₃, -COOR₃, - OCOOR₃, -OCON(R₃)₂, -CN, -NO₂, -SR₃, -OR₃, -N(R₃)₂, -CON(R₃)₂, - SO₂R₃, -SO₃H ou -S(=O)R₃, et en outre optionnellement interrompu par un ou plusieurs hétéroatomes identiques ou différents sélectionnés parmi S, O ou N, et/ou au moins un groupe sélectionné parmi -NH-CO- , -CO-NH- ou -N(C₁-C₈alkyl)-, lequel R₃ étant chacun indépendamment choisi parmi l'hydrogène ou - alkyle (C₁-C₈) ; ou
(ii) ledit agent chélateur est l'acide N,N'-bis[2-hydroxy-5-(carboxyéthyl)benzyl]éthylènediamine-N,N'-diacétique (HBED-CC) ou l'acide 1,4,7,10-tétraazacyclododécane-1,4,7,10-tétraacétique (DOTA), formant une liaison amide avec soit L, s'il est présent, ou R₂, et lorsque X est présent, formant une autre liaison amide avec L', s'il est présent, ou R₂'.

3. Le procédé selon la revendication 2, dans lequel :
(i) ledit lieur est un résidu d'acide aminé, ou un fragment peptidique constitué de 2 à 6 résidus d'acides aminés, ledit acide aminé étant chacun indépendamment l'acide 6-aminohexanoïque, l'acide 8-aminooctanoïque, la 1-naphthylalanine (1Nal), le 2-naphthylalanine (2Nal), ou l'acide 4-(aminométhyl)cyclohexane carboxylique (Amc), de préférence un résidu d'acide 6-aminohexanoïque ou d'acide 8-aminooctanoïque, ou une fraction de 1Nal-Amc, 2Nal-Amc, Amc-1Nal ou Amc-2Nal ; ou
(ii) ledit lieur est chacun indépendamment un alkylène (C₁-C₈), un alcénylène (C₂-C₈) ou un alcynylène (C₂-C₈), optionnellement substitué par un ou plusieurs groupes, chacun indépendamment étant choisi parmi halogène, -COH, -COOH, -OCOOH, -OCONH₂, -CN, -NO₂, - SH, -OH, -NH₂, -CONH₂, -SO₂H, -SO₃H ou -S(=O)H, et en outre optionnellement interrompu par un ou plusieurs hétéroatomes identiques ou différents, choisi parmi S, O ou N, et/ou au moins un groupe choisi parmi -NH-CO-, -CO-NH- ou -N(C₁-C₈alkyl)-.

4. Le procédé selon la revendication 1, dans lequel :
L et L' sont chacun indépendamment absents ou un lieur sélectionné parmi un résidu d'acide aminé formant une liaison peptidique avec R₂ ou R₂', respectivement, une fraction peptidique constituée de 2 à 6 résidus d'acides aminés et formant une liaison peptidique avec R₂ ou R₂', respectivement, alkylène (C₁-C₈), alcénylène (C₂-C₈) ou alcynylène (C₂-C₈), ledit alkylène (C₁-C₈), alcénylène (C₂-Cs) et alcynylène (C₂-C₈) est chacun indépendamment facultativement substitué avec un ou plusieurs groupes, chacun étant choisi indépendamment parmi halogène, -COR₃, -COOR₃, -OCOOR₃, -OCON(R₃)₂, -CN, -NO₂, -SR₃, -OR₃, - N(R₃)₂, -CON(R₃)₂, -SO₂R₃, -SO₃H ou -S(=O)R₃, et étant en outre optionnellement interrompu par un ou plusieurs hétéroatomes identiques ou différents sélectionnés parmi S, O ou N, et/ou au moins un groupe sélectionné parmi -NH-CO-, -CO-NH- ou -N(C₁-C₈alkyl)-, R₃ étant chacun indépendamment choisi parmi l'hydrogène ou -alkyle (C₁-C₈) ; et
ledit agent chélateur est HBED-CC ou DOTA, formant une liaison amide avec soit L, s'il est présent, soit R₂, et lorsque X est présent, formant une autre liaison amide avec soit L', s'il est présent, soit R₂'.

5. Le procédé selon la revendication 4, dans lequel L et L', s'ils sont présents, représentent chacun indépendamment un lieur choisi parmi un résidu d'acide aminé, un fragment peptidique constitué de 2 à 6 résidus d'acide aminé, un alkylène (C₁-C₈), un alcénylène (C₂-C₈) ou un alcynylène (C₂-C₈), ledit acide aminé étant chacun indépendamment l'acide 6-aminohexanoïque, l'acide 8-aminooctanoïque, 1Nal, 2Nal ou Amc ; et ledit alkylène (C₁-C₈), alcénylène (C₂-C₈) et alcynylène (C₂-C₈) est optionnellement substitué par un ou plusieurs groupes, chacun indépendamment étant choisi parmi halogène, -COH, -COOH, - OCOOH, -OCONH₂, -CN, -NO₂, -SH, -OH, -NH₂, -CONH₂, -SO₂H, -SO₃H ou - S(=O)H, et en outre optionnellement interrompu par un ou plusieurs hétéroatomes identiques ou différents sélectionnés parmi S, O ou N et/ou au moins un groupe choisi parmi -NH-CO-, -CO-NH- ou -N(C₁-C₈alkyle).

6. Le procédé selon la revendication 5, dans lequel L et L' représentent chacun indépendamment un résidu d'acide 6-aminohexanoïque ou d'acide 8-aminooctanoïque, ou une fraction de 1Nal-Amc, 2Nal-Amc, Amc-1Nal ou Amc-2Nal.

7. Le procédé selon la revendication 6, dans lequel (i) X est absent ; R₂ est un résidu lysine lié via son groupe α-amino au groupe -CO- adjacent ; et (a) L est l'acide 6-aminohexanoïque ; et B représente HBED-CC lié via un groupe carboxylique de celui-ci au groupe amino de l'acide 6-aminohexanoïque ; ou (b) L représente Amc-2Nal ou Ame-INal lié via le groupe carboxylique du 2Nal ou du 1Nal, respectivement, au groupe amino de la chaîne latérale de R₂ ; et B est DOTA lié via un groupe carboxylique de celui-ci au groupe amino de l'Amc ; ou (ii) X est présent ; R₂ et R₂' représentent chacun un résidu lysine lié via son groupe α-amino au groupe -CO- adjacent ; L et L' représentent chacun l'acide 6-aminohexanoïque ; et B est HBED-CC lié via un groupe carboxylique de celui-ci au groupe amino de L et via un autre groupe carboxylique de celui-ci au groupe amino de L'.

8. Le procédé selon la revendication 7, dans lequel ledit isotope radioactif est ⁶⁸Ga.

9. Le procédé selon l'une quelconque des revendications 1 à 8, dans lequel
(a) ledit flacon de réaction fourni à l'étape (i) est prétraité avec de l'HCl ; ou
(b) ledit agent tampon dans l'étape (i) est le formiate de sodium, l'ascorbate de sodium, l'acétate de sodium, l'hydroxyde de sodium ou le citrate de sodium ; ou
(c) la solution ajoutée à l'étape (ii) audit flacon de réaction est obtenue en éluant ledit isotope radioactif à partir d'un générateur d'isotopes radioactifs, immédiatement avant l'étape (ii), en utilisant ladite quantité prédéterminée de HCl comme solvant d'élution ; ou
(d) ledit mélange à l'étape (iii) est effectué par remuage ou secouage ; ou
(e) ledit isotope radioactif est ⁶⁸Ga, et la solution de l'étape (iii) est incubée pendant 2 à 10 minutes, de préférence 2 à 6 minutes, à température ambiante ; ou ledit isotope radioactif est ¹⁷⁷Lu ou ⁹⁰Y, et la solution de l'étape (iii) est incubée pendant environ 30 minutes ou plus à une température d'environ 100°C ou plus ; ou
(f) le pH de la solution à l'étape (iv) est ajusté à un pH compris entre 4,0 et 8,0, en ajoutant du formiate de sodium, de l'ascorbate de sodium, de l'acétate de sodium, de l'hydroxyde de sodium ou du citrate de sodium.

10. Un procédé de marquage d'un ligand PSMA avec ⁶⁸Ga, ledit procédé comprenant les étapes consistant à :
(i) fournir un flacon de réaction contenant environ 800 à 1800 mg de formiate de sodium et environ 10 µg dudit ligand PSMA, à la fois le formiate de sodium et ledit ligand PSMA étant séchés ;
(ii) ajouter une solution de ⁶⁸Ga dans environ 1,8 ml de HCl 0,1N audit flacon de réaction, de façon à obtenir une solution dudit ligand PSMA et dudit ⁶⁸Ga dans ledit HCl, ladite solution de ⁶⁸Ga dans de l'HCl étant obtenue à partir d'un générateur de ⁶⁸Ga, immédiatement avant cette étape, en utilisant ledit HCl comme solvant d'élution ;
(iii) mélanger la solution obtenue en (ii) puis l'incuber pendant 2 à 10 minutes, de préférence 2 à 6 minutes, à température ambiante, faisant ainsi réagir ledit ligand PSMA avec ledit ⁶⁸Ga et obtenir ledit ligand PSMA marqué par le ⁶⁸Ga ; et
(iv) optionnellement ajuster le pH de la solution dans ledit flacon de réaction à un pH compris dans une gamme de 4,0 à 8,0, en ajoutant environ 0,1 ml d'hydroxyde de sodium 1N,
ledit ligand PSMA répondant à la formule générale I' :
**I'** R₁-CO-R₂-L-B
dans laquelle
R₁ est un résidu d'acide glutamique lié via son groupe amino au groupe -CO-adjacent ; R₂ est un résidu lysine lié via son groupe α-amino au groupe -CO-adjacent ; et soit : (a) L est l'acide 6-aminohexanoïque lié via son groupe carboxylique au groupe amino de la chaîne latérale de R₂ ; et B représente HBED-CC lié via un groupe carboxylique de celui-ci au groupe amino de l'acide 6-aminohexanoïque ; ou (b) L représente Amc-2Nal ou Amc-lNal lié via le groupe carboxylique de 2Nal ou 1Nal, respectivement, au groupe amino de la chaîne latérale de R₂ ; et B est DOTA lié via un groupe carboxylique de celui-ci au groupe amino de l'Amc.

11. Le procédé de marquage d'un ligand PSMA par ¹⁷⁷Lu ou ⁹⁰Y, ledit procédé comprenant les étapes suivantes :
(i) fournir un flacon de réaction contenant du formiate de sodium et 10 µg dudit ligand PSMA, le formiate de sodium et ledit ligand PSMA étant, tant l'un que l'autre, séchés ;
(ii) ajouter une solution de ¹⁷⁷Lu ou ⁹⁰Y dans environ 0,5 ml de HCl 0,1N audit flacon de réaction, de façon à obtenir une solution dudit ligand PSMA et dudit ¹⁷⁷Lu ou ⁹⁰Y dans ledit HCl ; et
(iii) mélanger la solution obtenue en (ii) puis l'incuber pendant environ 30 minutes à une température d'environ 100°C ou plus, faisant ainsi réagir ledit ligand PSMA avec ledit ¹⁷⁷Lu ou ⁹⁰Y et obtenant ledit ligand PSMA marqué avec ¹⁷⁷Lu ou ⁹⁰Y,
ledit ligand PSMA étant de la formule générale I' :
**I'** R₁-CO-R₂-L-B
dans laquelle
R₁ est un résidu d'acide glutamique lié via son groupe amino au groupe -CO-adjacent ; R₂ est un résidu lysine lié via son groupe α-amino au groupe -CO-adjacent ; et L est du Amc-2Nal ou Amc-lNal lié via le groupe carboxylique du 2Nal ou du 1Nal, respectivement, au groupe amino de la chaîne latérale de R₂ ; et B est DOTA lié via un groupe carboxylique de celui-ci au groupe amino de l'Amc.
